# FASCICULE DE BREVET EUROPEEN

(11) **EP 3 642 135 B1**
(45) Date de publication et mention de la délivrance du brevet: **12.05.2021**
(21) Numéro de dépôt: 17748818.6
(22) Date de dépôt: 20.06.2017
(51) Int. Cl.: B65D 83/54, A61M 15/00

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(43) Date de publication de la demande: 29.04.2020
(73) Titulaire: APTAR France SAS, 27110 Le Neubourg (FR)
(72) Inventeur: LÉONÉ , Patrice, 27400 Acquigny (FR)
(74) Mandataire: CAPRI
(86) Numéro de dépôt international: PCT/FR2017/051626
(87) Numéro de publication internationale: WO 2018/234640

(56) Documents cités:
- EP-A1- 0 534 308
- EP-A1- 1 200 321
- WO-A1-99/19235
- US-A- 5 474 758
- US-A1- 2008 230 567

## Description

La présente invention concerne un dispositif de distribution de produit fluide.

Plus particulièrement, la présente invention concerne un dispositif de distribution de produit fluide pharmaceutique du type inhalateur à dose mesurée, communément appelé pMDI (pressurized Metered Dose Inhaler). Dans ce type de dispositif, le produit fluide pharmaceutique, qui contient un ou plusieurs produit(s) actif(s), est associé à un gaz propulseur et disposé dans un réservoir sous pression. De manière générale, le réservoir contient le produit fluide et le gaz propulseur, en particulier une formulation constituée d'un ou plusieurs principe(s) actif(s) en suspension et/ou en solution dans un gaz propulseur liquéfié, ainsi qu'éventuellement des excipients. Une valve doseuse est assemblée sur le réservoir et est actionnée pour distribuer une dose de produit fluide à chaque actionnement. Les inhalateurs de ce type comportent un corps externe dans lequel le réservoir peut coulisser, généralement axialement, pour actionner la valve et distribuer la dose de produit fluide à travers un orifice de distribution, généralement un embout buccal. Ce type de dispositif est bien connu dans l'état de la technique. Il existe de nombreux types de valves doseuses utilisables avec ce type d'inhalateur. De manière générale, une valve doseuse comporte un corps de valve dans lequel coulisse une soupape. Le corps de valve contient une chambre de dosage et lorsque la soupape est enfoncée, la chambre de dosage se vide à travers ladite soupape sous l'effet du gaz propulseur. Lorsqu'ensuite la soupape revient vers sa position de repos, une nouvelle dose est chargée dans la chambre de dosage. De manière connue, ce type d'inhalateur pMDI comporte un ou plusieurs élément(s) d'étanchéité. Les éléments d'étanchéité assurent l'étanchéité à différents endroits, et de manière classique, il existe généralement un joint de col, qui est interposé entre la valve doseuse, le réservoir et la bague de fixation qui sert à fixer la valve sur le réservoir. Par ailleurs, la valve elle-même comporte un ou coopère(nt) avec la soupape lorsque celle-ci est au repos et/ou lorsqu'elle se déplace vers sa position actionnée. Dans les valves les plus courantes, la valve comporte généralement deux joints appelés « joints internes » contre lesquels la soupape va coulisser de manière étanche lors de l'actionnement. Ces différents éléments d'étanchéité sont donc susceptibles d'être en contact avec le produit actif contenu dans le produit fluide à distribuer. Ils sont également en contact avec le gaz propulseur. Généralement, ces éléments d'étanchéité sont réalisés en matériau élastomère, du type EPDM, nitrile, polychloroprène, etc. Tous ces matériaux sont plus ou moins performants selon les propriétés considérées, et présentent tous certains inconvénients. En particulier, ils sont susceptibles d'interagir avec le produit actif et/ou le gaz propulseur. Il est donc souhaitable de trouver des matériaux pour réaliser ces éléments d'étanchéité qui interagissent le moins possible avec ledit produit actif et/ou ledit gaz propulseur, tout en étant facile à fabriquer et à assembler, pour s'adapter aux chaînes de montage à haute cadence typiques pour ces dispositifs d'inhalateur.

Le document WO2010096106 décrit un matériau appelé CBC (Cyclic Block Copolymer) ayant principalement des applications optiques, notamment la fabrication de parties d'écrans LCD. D'autres applications connues du CBC comprennent des applications dans le domaine alimentaire et dans le domaine médical, notamment la réalisation de parties de seringues ou de poches transparentes destinées à contenir des fluides. Ce matériau CBC n'a toutefois jamais été utilisé en tant qu'élément d'étanchéité dans des dispositifs du type pMDI, en particulier en contact avec des gaz propulseur, qui ont une action très agressive sur les matériaux constitutifs des éléments d'étanchéité. Or, il a été constaté de manière surprenante que ce matériau CBC s'avère particulièrement bénéfique et adapté à être utilisé dans ces applications de valve doseuse fonctionnant avec des gaz propulseur, notamment des gaz du type HFA.

Les documents US5474758 et WO99/19235 décrivent d'autres dispositifs de l'état de la technique.

La présente invention a donc pour but de fournir un dispositif de distribution de produit fluide qui ne reproduit pas les inconvénients susmentionnés.

Plus particulièrement, la présente invention a pour but de fournir un dispositif de distribution de produit fluide qui améliore les propriétés des éléments d'étanchéité utilisées dans le dispositif, et qui limite les interactions néfastes entre lesdits éléments d'étanchéité et le produit fluide et/ou le gaz propulseur avec lesquels il est en contact.

La présente invention a aussi pour but de fournir un dispositif de distribution de produit fluide qui soit simple et peu coûteux à fabriquer et à assembler.

La présente invention a donc pour objet un dispositif de distribution de produit fluide comprenant un corps pourvu d'un orifice de distribution, un réservoir contenant du produit fluide et un gaz propulseur, une valve doseuse assemblée sur ledit réservoir, ledit réservoir étant déplaçable dans ledit corps pour actionner la valve doseuse et distribuer une dose de produit fluide à travers ledit orifice de distribution, ladite valve doseuse comportant une soupape coulissant dans ladite valve doseuse lors de l'actionnement, ledit dispositif comportant au moins un élément d'étanchéité pour former une étanchéité au fluide, au moins un élément d'étanchéité dudit dispositif comportant du CBC (Cyclic Block Copolymer).

Avantageusement, ladite valve doseuse est assemblée sur ledit réservoir avec interposition d'un joint de col.

Avantageusement, ladite valve doseuse comporte au moins un joint interne coopérant de manière étanche avec ladite soupape.

Avantageusement, ladite valve doseuse comporte un joint interne supérieur et un joint interne inférieur définissant entre eux une chambre de dosage de ladite valve doseuse.

Avantageusement, ledit joint de col et/ou ledit joint interne supérieur et/ou ledit joint interne inférieur comporte(nt) du CBC.

Avantageusement, ledit au moins un élément d'étanchéité est constitué de CBC.

Avantageusement, ledit produit fluide est un produit fluide pharmaceutique comportant au moins un produit actif.

Avantageusement, ledit gaz propulseur comporte des gaz HFA, notamment du type HFA 134a et/ou HFA 227 et/ou HFA 152a.

Avantageusement, une bague est associée à la valve doseuse, au moins un élément d'étanchéité en CBC étant surmoulé sur une partie de ladite valve doseuse et/ou de ladite bague.

Ces avantages et d'autres de la présente invention apparaitront plus clairement au cours de la description détaillée suivante, faite en référence aux dessins joints, donnés à titre d'exemples non limitatifs, et sur lesquels :
- la figure 1 est une vue schématique en section transversale d'un dispositif de distribution de produit fluide du type inhalateur à dose mesurée (pMDI),
- la figure 2 est une vue schématique en section transversale d'un réservoir sur lequel est assemblée une valve doseuse, selon un mode de réalisation avantageux de la présente invention, et
- la figure 3 illustre la structure chimique du CBC et son procédé de fabrication par hydrogénation du SBS.

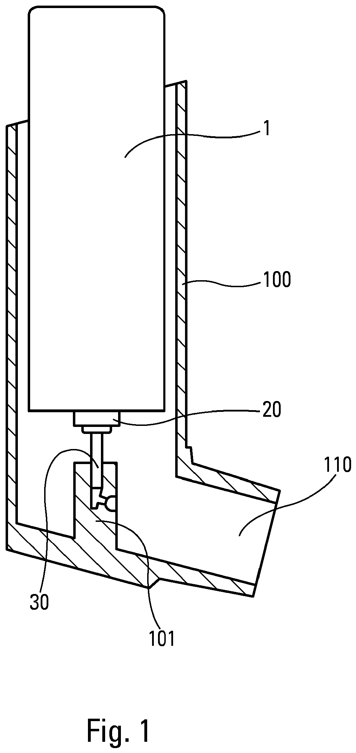

En se référant à la figure 1, il est décrit un inhalateur à dose mesurée appelé généralement pMDI, qui classiquement comporte un corps externe 100 pourvu d'un orifice de distribution 110, généralement un embout buccal. A l'intérieur de ce corps est disposé un réservoir 1 sur lequel est montée une valve doseuse 20. Une soupape 30 coulisse dans ladite valve doseuse 20 pour distribuer une dose de produit fluide à chaque actionnement. Le corps 100 comporte un puits 101 qui reçoit la soupape 30 et qui crée un passage de liaison entre la sortie de la soupape 30 et ledit orifice de distribution 110. De manière classique, pour actionner un tel dispositif, l'utilisateur appuie sur le fond du réservoir 1 pour enfoncer celui-ci axialement à l'intérieur du corps 100, ce qui provoque le coulissement étanche de la soupape 30 vers l'intérieur de la valve doseuse 20 provoquant ainsi la distribution d'une dose de produit fluide. A l'intérieur du réservoir, le produit fluide, qui contient généralement un ou plusieurs produit(s) actif(s), est associé à un gaz propulseur, de préférence un gaz du type HFA, par exemple HFA 134a (1,1,1,2-tétrafluoroéthane) et/ou HFA 227 (1,1,1,2,3,3,3-heptafluoropropane) et/ou HFA 152a (1,1-difluoroéthane).

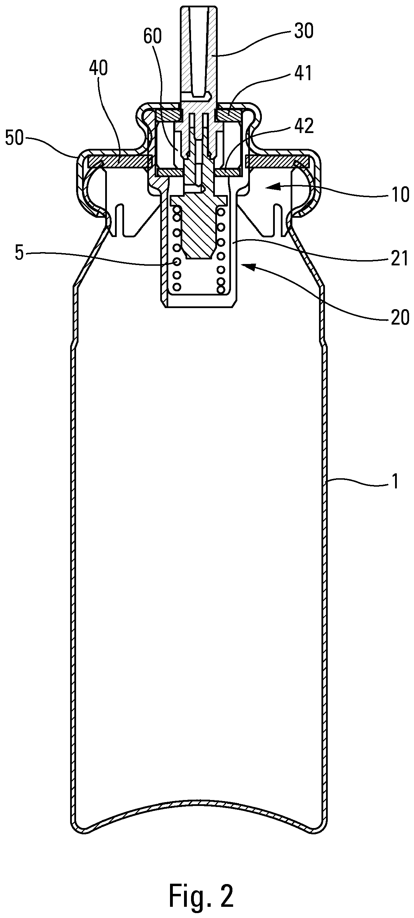

La figure 2 représente une valve doseuse selon un mode de réalisation particulier qui est avantageux. Il est entendu que la présente invention n'est pas limitée à ce type de valve doseuse, mais qu'elle s'applique à tout type de valve doseuse utilisable dans les pMDI.

Une valve doseuse 20 est assemblée sur le réservoir 1 comme visible sur la figure 2. Cet assemblage peut être réalisé au moyen d'une bague de fixation 50, qui en l'occurrence est une bague sertie, mais qui pourrait également être une bague encliquetable ou vissable. La valve doseuse 20 comporte classiquement un corps de valve 21 à l'intérieur duquel coulisse une soupape 30. Cette soupape 30 est sollicitée par un ressort 5 vers sa position de repos.

De manière connue, un joint d'étanchéité 40, appelé « joint de col », est interposé entre la bague de fixation 50 et le col du réservoir 1 lors de l'assemblage de la valve doseuse 20 sur le réservoir 1, pour assurer l'étanchéité au niveau du col du réservoir.

De plus, la valve doseuse comporte au moins un joint d'étanchéité interne 41, 42 qui coopère de manière étanche avec la soupape 30. Dans l'exemple représenté sur la figure 2, la valve comporte un joint interne supérieur 41 et un joint interne inférieur 42, les termes « inférieur » et « supérieur » se référant à l'orientation de la figure 2, c'est-à-dire avec la valve 20 disposée au-dessus du réservoir 1. Entre ces deux joints internes 41, 42 est définie une chambre de dosage 60 et lorsque la soupape 30 est enfoncée dans le corps de valve 21, le contenu de cette chambre de dosage 60 est expulsé à travers la soupape 30, de manière classique.

Une bague 10 peut être interposée entre le joint de col 40 et le corps de valve 21 pour limiter les contacts entre le produit actif et le joint de col 40, mais également pour limiter le volume mort dans cet endroit du dispositif. Cette bague 10, lorsque présente, peut être de forme et de matériau quelconque approprié.

Selon l'invention, au moins un des éléments d'étanchéité, c'est-à-dire au moins un du joint de col 40, du joint interne supérieur 41 et du joint interne inférieur 42 comporte du CBC (Cyclic Block Copolymer).

De préférence, les trois joints ci-dessus sont réalisés avec ce matériau.

Avantageusement, le CBC forme le seul matériau de base, mais on pourrait envisager de réaliser un alliage de CBC avec un ou plusieurs autres matériaux, notamment du type élastomère.

Le CBC est notamment fabriqué et commercialisé par la société USI CORPORATION, notamment sous la marque Puratran™.

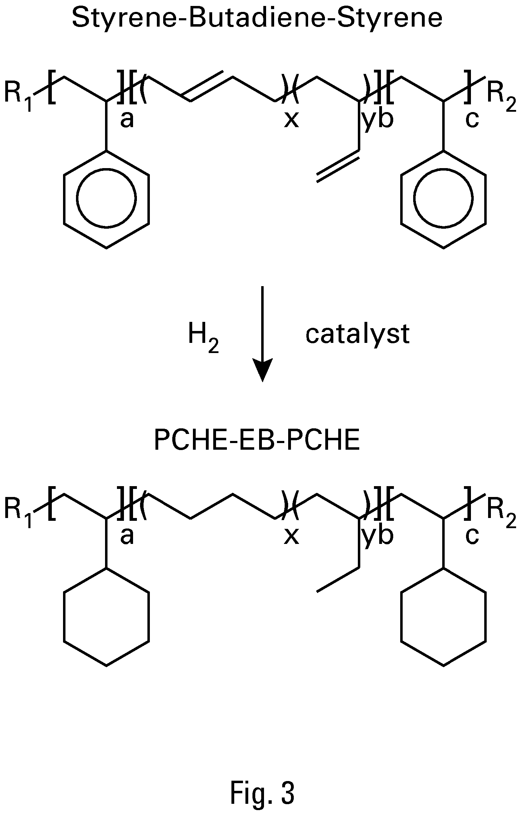

Le CBC est un matériau obtenu à partir d'une hydrogénation totale du SBS (Styrène Butadiène Styrène), comme illustré sur la figure 3.

Le CBC n'est donc pas un mélange ou un alliage de matériaux élastomères classiques, mais c'est un matériau en tant que tel.

Sa structure chimique lui confère des propriétés intéressantes, telles que : transparence, faible absorption d'humidité, grande pureté, résistance thermique élevée, résistance aux UV, résistance aux acides et aux bases.

De par son procédé de fabrication, il est possible d'obtenir différents grades allant d'une grande rigidité à une grande souplesse, similaire aux élastomères thermoplastiques (TPE).

L'utilisation du CBC en tant qu'élément d'étanchéité d'une valve doseuse présente de nombreux avantages, notamment par rapport à des matériaux élastomères plus classiques tels que le nitrile, l'EPDM ou le polychloroprène:
1) Le fait que le CBC ne contienne pas de double liaison lui donne une neutralité beaucoup plus importante que les autres élastomères; ceci génère moins d'interactions avec les principes actifs.
2) Cette saturation lui confère aussi une bonne résistance au vieillissement; ceci est important notamment pour le stockage, pour avoir une durée de vie des valves la plus longue possible.
3) Sa grande pureté lui donne un niveau d'extractibles très bas et là encore limite les risques de dégradation du principe actif.
4) Sa bonne résistance aux produits chimiques assure une bonne tenue dans les propulseurs (avec et sans alcool), notamment ceux du type HFA, et limite les problèmes de dégradation rencontrés avec ces produits agressifs.
5) Sa faible absorption d'humidité lui confère de très bonnes propriétés barrière la vapeur d'eau et améliorera l'étanchéité dans la valve.

Il s'est ainsi avéré que l'utilisation de CBC pour réaliser les éléments d'étanchéité permet d'améliorer le fonctionnement des valves doseuses, de diminuer les interactions avec le produit actif et/ou le gaz propulseur, tout en rendant moins difficile ou compliqué et donc moins coûteux la fabrication et l'assemblage des valves et des inhalateurs dans lesquels ces valves sont utilisées.

Bien que la présente invention ait été décrite en référence à un mode de réalisation avantageux de celle-ci, il est entendu qu'elle n'est pas limitée par celui-ci mais que toutes modifications utiles peuvent y être apportées sans sortir du cadre de la présente invention tel que défini par les revendications annexées.

## Revendications

1. Dispositif de distribution de produit fluide comprenant un corps (100) pourvu d'un orifice de distribution (110), un réservoir (1) contenant du produit fluide et un gaz propulseur, une valve doseuse (20) assemblée sur ledit réservoir (1), ledit réservoir (1) étant déplaçable dans ledit corps (100) pour actionner la valve doseuse (20) et distribuer une dose de produit fluide à travers ledit orifice de distribution (110), ladite valve doseuse (20) comportant une soupape (30) coulissant dans ladite valve doseuse (20) lors de l'actionnement, ledit dispositif comportant au moins un élément d'étanchéité (40, 41, 42) pour former une étanchéité au fluide, **caractérisé en ce qu'**au moins un élément d'étanchéité (40, 41, 42) dudit dispositif comporte du CBC (Cyclic Block Copolymer).

2. Dispositif selon la revendication 1, dans lequel ladite valve doseuse (20) est assemblée sur ledit réservoir (1) avec interposition d'un joint de col (40).

3. Dispositif selon la revendication 1 ou 2, dans lequel ladite valve doseuse (20) comporte au moins un joint interne (41, 42) coopérant de manière étanche avec ladite soupape (30).

4. Dispositif selon la revendication 3, dans lequel ladite valve doseuse (20) comporte un joint interne supérieur (41) et un joint interne inférieur (42) définissant entre eux une chambre de dosage (60) de ladite valve doseuse (20).

5. Dispositif selon la revendication 2 et selon l'une des revendications 3 ou 4, dans lequel ledit joint de col (40) et/ou ledit joint interne supérieur (41) et/ou ledit joint interne inférieur (42) comporte(nt) du CBC.

6. Dispositif selon l'une quelconque des revendications précédentes, dans lequel ledit au moins un élément d'étanchéité (40, 41, 42) est constitué de CBC.

7. Dispositif selon l'une quelconque des revendications précédentes, dans lequel ledit produit fluide est un produit fluide pharmaceutique comportant au moins un produit actif.

8. Dispositif selon l'une quelconque des revendications précédentes, dans lequel ledit gaz propulseur comporte des gaz HFA, notamment du type HFA 134a et/ou HFA 227 et/ou HFA 152a.

9. Dispositif selon l'une quelconque des revendications précédentes, dans lequel une bague (10) est associée à la valve doseuse (20), au moins un élément d'étanchéité (40, 41, 42) en CBC étant surmoulé sur une partie de ladite valve doseuse (20) et/ou de ladite bague (10).

## Patentansprüche

1. Vorrichtung zur Abgabe eines Fiuidprodukts, umfassend einen mit einer Abgabeöffnung (110) versehenen Körper (100), einen ein Fluidprodukt und ein Treibgas enthaltenden Vorratsbehälter (1), ein an dem Vorratsbehälter (1) angebrachtes Dosierventil (20), wobei der Vorratsbehälter (1) zur Betätigung des Dosierventils (20) und zur Abgabe einer Dosis Fluidprodukt über die Abgabeöffnung (110) in dem Körper (100) bewegbar ist, wobei das Dosierventil (20) ein Ventil (30) umfasst, das bei Betätigung in das Dosierventil (20) gleitet, wobei die Vorrichtung mindestens ein Dichtungselement (40, 41, 42) zur Bildung einer Fluiddichtung umfasst,
**dadurch gekennzeichnet, dass** mindestens ein Dichtelement (40, 41, 42) der Vorrichtung CBC (Cyclic Block Copolymer) umfasst.

2. Vorrichtung nach Anspruch 1, bei der das Dosierventil (20) unter Zwischenschaltung einer Halsdichtung (40) auf dem Behälter (1) montiert ist.

3. Vorrichtung nach Anspruch 1 oder 2, bei der das Dosierventil (20) mindestens eine innenliegende Dichtung (41, 42) aufweist, die abdichtend mit dem Ventil (30) zusammenwirkt.

4. Vorrichtung nach Anspruch 3, bei der das Dosierventil (20) eine obere innenliegende Dichtung (41) und eine untere innenliegende Dichtung (42) aufweist, zwischen denen eine Dosierkammer (60) des Dosierventils (20) ausgebildet ist.

5. Vorrichtung nach Anspruch 2 sowie nach einem der Ansprüche 3 oder 4, bei der die Halsdichtung (40) und/oder die obere innenliegende Dichtung (41) und/oder die untere innenliegende Dichtung (42) CBC enthält bzw. enthalten.

6. Vorrichtung nach einem der vorhergehenden Ansprüche, bei der das mindestens eine Dichtelement (40, 41, 42) aus CBC besteht.

7. Vorrichtung nach einem der vorhergehenden Ansprüche, bei der es sich bei dem Fluidprodukt um ein pharmazeutisches Fluidprodukt handelt, das mindestens einen Wirkstoff enthält.

8. Vorrichtung nach einem der vorhergehenden Ansprüche, bei der das Treibgas HFA-Gase umfasst, insbesondere vom Typ HFA 134a und/oder HFA 227 und/oder HFA 152a.

9. Vorrichtung nach einem der vorhergehenden Ansprüche, bei der dem Dosierventil (20) ein Ring (10) zugeordnet ist, wobei an einem Teil des Dosierventils (20) und/oder des Rings (10) mindestens ein Dichtelement (40, 41, 42) aus CBC angeformt ist.

## Claims

1. A fluid dispenser device comprising: a body (100) that is provided with a dispenser orifice (110), a reservoir (1) containing fluid and a propellant gas, and a metering valve (20) that is assembled on said reservoir (1), said reservoir (1) being movable in said body (100) so as to actuate the metering valve (20) and dispense a dose of fluid through said dispenser orifice (110), said metering valve (20) including a valve member (30) that slides in said metering valve (20) during actuation, said device further comprising at least one sealing element (40, 41, 42) so as to form a leaktight seal, the device being **characterized in that** at least one sealing element (40, 41, 42) of said device comprises Cyclic Block Copolymer (CBC).

2. A device according to claim 1, wherein said metering valve (20) is assembled on said reservoir (1) with a neck gasket (40) interposed therebetween.

3. A device according to claim 1 or claim 2, wherein said metering valve (20) includes at least one internal gasket (41, 42) that co-operates in leaktight manner with said valve member (30).

4. A device according to claim 3, wherein said metering valve (20) includes an upper internal gasket (41) and a lower internal gasket (42) defining between them a metering chamber (60) of said metering valve (20).

5. A device according to claim 2 and according to claim 3 or claim 4, wherein said neck gasket (40) and/or said upper internal gasket (41) and/or said lower internal gasket (42) comprise(s) CBC.

6. A device according to any preceding claim, wherein said at least one sealing element (40, 41, 42) is constituted by CBC.

7. A device according to any preceding claim, wherein said fluid is a pharmaceutical fluid containing at least one active substance.

8. A device according to any preceding claim, wherein said propellant gas comprises HFA gases, in particular of the HFA 134a and/or HFA 227 and/or HFA 152a type.

9. A device according to any preceding claim, wherein a ring (10) is associated with the metering valve (20), at least one sealing element (40, 41, 42) made of CBC being over-molded on a portion of said metering valve (20) and/or of said ring (10).
